# EUROPÄISCHE PATENTANMELDUNG

(11) **EP 0 598 460 A2**
(43) Veröffentlichungstag der Anmeldung: **25.05.1994**
(21) Anmeldenummer: 93250251.1
(22) Anmeldetag: 16.09.1993
(51) Int. Cl.: G01N 31/22, G01N 21/17, G01N 33/18

(54) **Verfahren zur Analyse von Stoffen und Messzelle zur Durchführung des Verfahrens**

(30) Priorität: 21.09.1992 DE 4231892
(71) Anmelder: INSTITUT FÜR BIOPROZESS- UND ANALYSENMESSTECHNIK e.V., D-37308 Heiligenstadt (DE); JOHANNISTHALER FORSCHUNGSTECHNIK GmbH, D-12489 Berlin (DE)
(72) Erfinder: Beckmann,Dieter, D-37308 Heilingenstadt (DE); Gruber,Ronald, D-37308 Geisleden (DE); Peschel,Klaus Dr., 10369 Berlin (DE); Thiede, Günther Dr., 12559 Berlin (DE); Weihert,Michael, 10405 Berlin (DE)
(74) Vertreter: Maikowski, Michael, Dipl.-Ing. Dr.

(57) **Zusammenfassung**

Die Anmeldung betrifft ein Verfahren und eine Anordnung zur Analyse von Stoffen unter Verwendung von lebenden biologischen Materialien. Erfindungsgemäß werden biologische Materialien als Indikatoren verwendet, deren photoakustisch meßbare Absorption sich unter dem Einfluß der zu analysierenden Stoffe verändert. Hierzu befindet sich das biologische Material in einem Küvettenraum, der die Gewinnung eines photoakustischen Signales gestattet und zugleich Reaktionsraum für den zu analysierenden Stoff mit dem biologischen Material ist. Das Verfahren und die Anordnung sind für die Feststellung von Schadstoffen sowohl in kleinsten Konzentrationen als auch bei Feldeinsätzen geeignet, und die Meßergebnisse sind für Prozeßsteuerungen verwendbar.

## Beschreibung

Die Erfindung betrifft ein Verfahren gemäß dem Oberbegriff des Anspruchs 1 und eine Meßzelle zur Durchführung des Verfahrens.

Die Erfindung bezieht sich insbesondere auf die Analyse von festen, wassergelösten und gasförmigen Stoffen, insbesondere von Schadstoffen, wie z.B. Ozon, SO₂, Dioxine, CO, CO₂, HCN, HCL und NH₃.

Für die Analyse von festen, flüssigen und gasförmigen Schadstoffen sind die unterschiedlichsten Verfahren bekannt. So ist es bekannt, entsprechende Analysen naßchemisch durchzuführen. Diese Verfahren erlauben jedoch nicht, kleinste Stoffmengen zu ermitteln. Ein weiterer Nachteil besteht darin, daß die Analyse eine gewisse Zeit in Anspruch nimmt, die die Ausnutzung dieser Verfahren für Prozeßsteuerungen ausschließt.

Weiterhin sind physikalische Analyse-Verfahren bekannt. Hierbei kommen für die genannten Stoffe insbesondere die spektroskopischen Verfahren in Betracht. Allgemein besteht bei den spektroskopischen Verfahren ebenfalls der Nachteil, daß kleinste Stoffmengen kaum erfaßbar sind.

Bekannt sind auch bestimmte biologische Analyse-Verfahren. Dabei werden Lebewesen, wie Mikroorganismen oder auch höhere Lebewesen, wie Fische, in die zu untersuchende Substanz eingebracht. Aus der Änderung des Verhaltens dieser Lebewesen lassen sich Rückschlüsse auf enthaltene Schadstoffe ziehen. Der Nachteil dieser Verfahren besteht darin, daß eine selektive Aussage über die Art der Schadstoffe nur schwer möglich ist. In der Regel ist nur eine Aussage darüber möglich, ob Schadstoffe vorhanden sind oder nicht. Ein weiterer Nachteil besteht darin, daß diese Aussagen nur visuell wahrgenommen werden können, so daß eine Einbindung dieser Verfahren in Prozeßsteuerungen unmöglich ist.

Weiterhin sind auch enzymatische Analyse-Verfahren bekannt. Dabei werden Enzyme mit den zu messenden Substanzen in Kontakt gebracht. Die anschließende Reaktion wird durch entsprechende bekannte Methoden verfolgt, um damit die Konzentration der Substanz zu ermitteln.

Der Nachteil dieser Analyse-Verfahren besteht darin, daß die Enzyme für Feldeinsätze nicht geeignet, sondern nur im Labor verwendbar sind. Weiterhin sind enzymatische Maßmethoden nur für in Wasser gelöste Stoffe und unter eng begrenzten physikalischen Bedingungen (Temperatur, Druck, ph-Wert) einsetzbar.

Der Erfindung liegt die Aufgabe zugrunde, die Analyse von festen, wassergelösten und gasförmigen Stoffen mit Hilfe lebender biologischer Materialien so zu verbessern, daß einzelne Stoffe und Stoffgruppen in geringsten Konzentrationen auch bei Feldeinsätzen ermittelt werden können und ein für Prozeßsteuerungen verwendbares Meßergebnis erzielbar ist.

Erfindungsgemäß wird das mit einem Verfahren gemäß der technischen Lehre des Anspruchs 1 erreicht.

Danach werden bei einem Verfahren zur Analyse von Stoffen unter Verwendung von lebenden biologischen Materialien erfindungsgemäß biologische Materialien als Indikatoren verwendet, deren photoakustisch meßbare Absorption sich unter dem Einfluß der zu analysierenden Stoffe verändert.

Dabei kann einerseits biologiches Material verwendet werden, dessen photoakustisch meßbare Absorption sich selektiv unter dem Einfluß einzelner Stoffe verändert. Andererseits kann biologisches Material verwendet werden, dessen photoakustisch meßbare Absorption sich selektiv unter dem Einfluß bestimmter Stoffgruppen verändert.

Eine zweckmäßige Ausführungsform des Verfahrens ist dadurch gekennzeichnet, daß in einem ersten Schritt der biologische Indikator ohne den zu analysierenden Stoff photoakustisch vermessen und das Ergebnis zwischengespeichert wird. Anschließend wird in einem zeiten Schritt der biologische Indikator mit dem zu analysierenden Stoff versetzt. In einem weiteren Schritt wird der mit dem zu analysierenden Stoff versetzte Indikator photoakustisch vermessen und dieses Meßergebnis wird anschließend mit dem zwischengespeicherten Meßergebnis verglichen. Das daraus resultierende Meßergebnis dient als Maß für die zu untersuchende Stoffmenge.

Als biologische Materialien können z.B. Rhodopsin-Moleküle zum Nachweis von HCN, HCL und Hämoglobin-Moleküle zum Nachweis von CO verwendet werden. Weiterhin können pflanzliche Zellen, z.B. einzellige Algen (Chlorella), zum Nachweis von Dioxinen und tierische Zellen, z.B. Blutzellen, zum Nachweis von CO₂, Ozon, HCN verwendet werden.

Als biologisches Material können auch Flechten, Blätter und Mikroorganismen, wie z.B. Halobakterien, Hefen oder Pilze, verwendet werden.

Neben der Ermittlung von Stoffen oder Stoffgruppen kann das Verfahren zur Auswahl (Screening) biologischer Materialien verwendet werden, indem ein Stoff bzw. eine Stoffgruppe in bekannter Konzentration und Menge mit dem biologischen Material in Kontakt gebracht wird und aus der Reaktion des biologischen Materials auf dieses geschlossen werden kann.

Eine Meßzelle zur Durchführung des Verfahrens weist einen Hohlraum auf, in dem sich der Indikator befindet, der die Gewinnung eines photoakustischen Signales gestattet und zugleich Reaktionsraum für den Schadstoff ist.

Eine spezielle Ausführungsform der Meßzelle ist dadurch gekennzeichnet, daß sie zweiteilig ist, aus einem Unter- und einem Oberteil besteht, wobei das Unterteil eine flache Schale ist, die sich zur Züchtung des biologischen Materials eignet.In diese Schale können auch auf Trägern befindliche Algenkulturen eingebracht werden. Das Oberteil besteht aus optisch durchsichtigem Glas, hat einen Einfüll- und einen Austrittsstutzen und weist eine Öffnung für das akustische Ankoppeln eines photoakustischen Sensors auf.

Es ist zweckmäßig, daß Ober- und Unterteil zur Gewährleistung der erforderlichen Dichtigkeit eingeschliffen und daß der Einfüll- und der Austrittsstutzen gasdicht verschließbar sind und daß die Unterkante des Einfüllstutzens über die Unterkante des Oberteils in den Probenraum ragt.

Es ist weiterhin zweckmäßig, daß mindestens der Verschluß eines Stutzens, vorzugsweise des Einfüllstutzens, zur definierten Einleitung des Schadstoffes während des gesamten Analysenvorganges zu öffnen ist.

Mit dieser Verfahrensweise und der zugehörigen Meßzelle wird eine hohe Empfindlichkeit und niedrige Nachweisgrenze sowohl im Labor als auch im Feldeinsatz erzielt. Weiterhin erlaubt dieses Verfahren eine Echtzeitverarbeitung und eröffnet damit die Möglichkeit des Einsatzes in Prozessen.

Die Erfindung soll in einem Ausführungsbeispiel anhand von Zeichnungen erläutert werden.

Es zeigen:

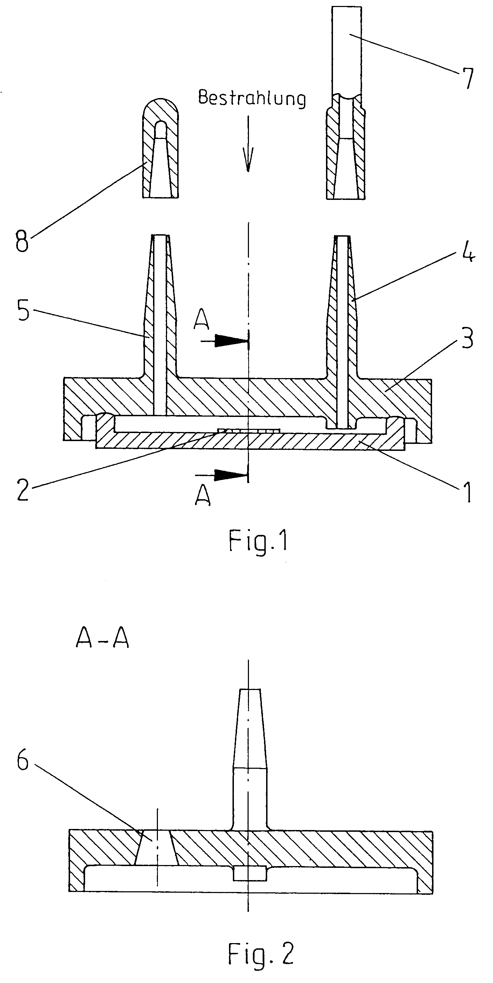

- Fig. 1: einen Schnitt durch eine erfindungsgemäße Meßzelle;
- Fig. 2: einen um 90 Grad gegenüber der Fig. 1 gedrehten Schnitt entlang der Linie A - A ohne die Teile 1 und 2 aus der Fig. 1;

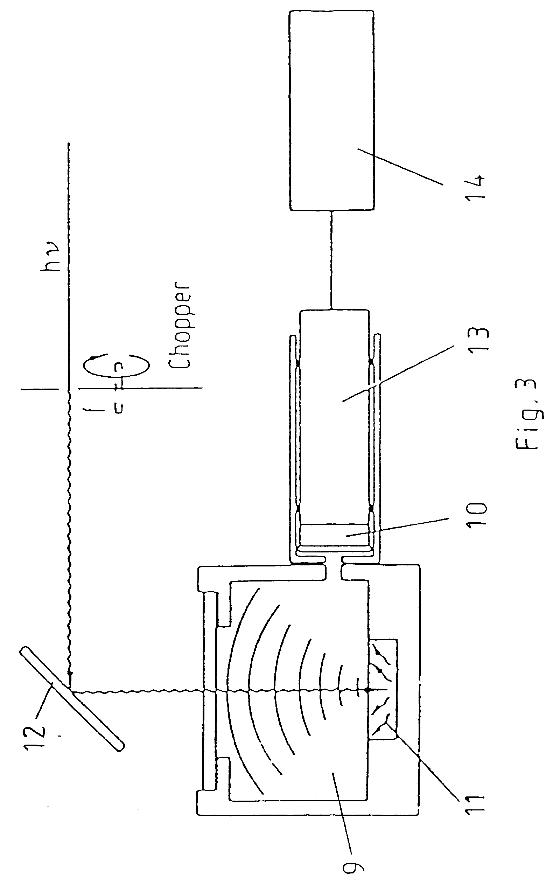

- Fig. 3: den prinzipiellen Meßaufbau;
- Fig. 4: Meßergebnisse mit einzelligen Algen vom Stamm Chlorella kesslerii.

Die Meßzelle hat eine untere Schale 1 für die Aufnahme der biologischen Materialien. Das biologische Material kann z.B. auf einem Objektträger 2 aufgebracht sein. Auf der unteren Schale 1 sitzt ein Deckel 3 aus optisch durchsichtigem Glas, die beide eingeschliffen sind, um die erforderliche Dichtigkeit zu gewährleisten. Der Deckel hat einen Einfüllstutzen 4 und einen Austrittsstutzen 5 sowie eine Öffnung 6, in deren Bereich ein photoakustischer Sensor angeordnet wird. Dem Einfüllstutzen 4 ist ein Schlauchanschluß 7 und dem Austrittsstutzen 5 ein Verschluß 8 zugeordnet.

Das biologische Material, das als Indikator für die Bestimmung eines Schadstoffs geeignet ist, wird auf einen festen Träger oder auf einen geeigneten Nährboden, z.B. Agar-Agar, gebracht und anschließend kann die untere Schale mit einer geeigneten Flüssgkeit aufgefüllt und mit dem Deckel 3 verschlossen werden. In diesem Zustand, d.h. ohne Einwirkung von Schadstoff, wird eine erste photoakustische Messung durchgeführt.

Durch den Einfüllstutzen 4 wird anschließend fester, flüssiger oder gasförmiger Schadstoff oder ein Gemisch dieser Schadstoffe eingeleitet. Sowohl dieser Einfüllstutzen 4 als auch der Austrittsstutzen 5 durch den Verschluß 8 werden dann verschlossen. Nach einer vorbestimmten Einwirkzeit des Schadstoffes auf den Indikator wird dieser durch den Deckel 3 mit geeignetem Licht bestrahlt und der auftretende photoakustische Effekt in bekannter Weise gemessen.

Die Anordnung gestattet auch während des gesamten Analysevorgangs das Einleiten von Schadstoffen über den Einfüllstutzen. So können die Schadstoffzuführungen und die photoakustische Messung abwechselnd erfolgen, um so schrittweise an ein quantitativ verwertbares Meßergebnis zu gelangen.

Die typische Vorrichtung für photoakustische Messungen besteht aus einer gasdichten Zelle 9, in der ein empfindliches Mikrophon 10 und die Probe 11 eingeschlossen sind. Die Probe wird über einen Umlenkspiegel 12 mit periodisch moduliertem Licht der Wellenlänge Lambda bestrahlt. Absorbiert die Probe 11 Licht dieser Wellenlänge, dann werden Elektronen in höhere Energiezustände angeregt.

Nach kurzer Zeit (< 10⁻⁹ s) kehren die angeregten Elektronen über Strahlungsprozesse und/oder strahlungslose Desaktivierung in den Grundzustand zurück. Der photoakustische Effekt beruht allein auf diesen strahlungslosen Vorgängen. In den Bereichen, in denen die Probe Licht absorbiert, erzeugen die strahlungslosen Prozesse eine periodische Erwärmung mit der Modulationsfrequenz, die sich innerhalb der Probe in alle Richtungen ausbreitet.

Der Teil der Wärme, der an die Probenoberfläche gelangt, gibt seine thermische Energie an die umgebende Luft ab. Eine sehr dünne Luft-Grenzschicht (<0,5 mm) reagiert nun durch periodisches Expandieren und Kontrahieren auf den mit der Modulationsfrequenz erfolgenden Erwärmungs- und Abkühlvorgang. Diese dünne Luftschicht erzeugt wie ein akustischer Kolben in der angrenzenden Luftsäule Druckschwankungen, die vom Mikrophon 10 als photoakustisches Signal aufgenommen werden. Die daraus abgeleiteten elektischen Signale des Mikrophons 10 werden über einen Vorverstärker 13 einer Signalverarbeitungseinheit 14 zugeführt. Wird das Licht der Wellenlänge Lambda nicht absorbiert, tritt kein photoakustisches Signal auf.

Das photoakustische Signal korreliert also mit dem optischen Spektrum und damit ergibt eine Aufzeichnung des Mikrophonsignals über der Wellenlänge das Absorptionsspektrum der Probe.

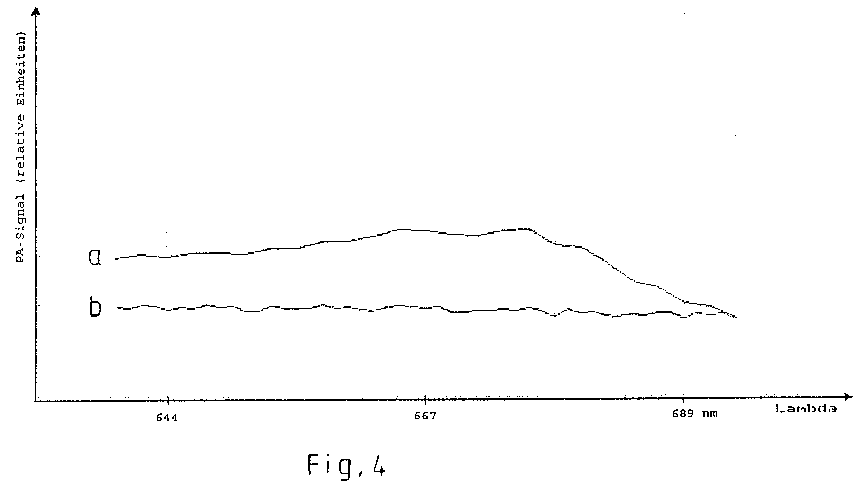

Mit einer Vorrichtung, die den beschriebenen prinzipiellen Aufbau hat, sind lebende einzellige Algen vom Stamm Chlorella kesslerii untersucht worden. Sie wurden auf Filterpapier gebracht und photoakustisch vermessen. Es ergab sich die Meßkurve a der Fig. 4.

Anschließend wurden diese Algen mit einer wäßrigen Schadstofflösung (2,4-Dichlorphenol, 0,1%ig) versetzt. Nach ca. 10 Minuten Einwirkzeit wurden die Algen photoakustisch vermessen. Es ergab sich die Meßkurve b der Fig. 4. Diese Kurven sind deutlich voneinander zu unterscheiden, d.h. es ist möglich, den Schadstoff nachzuweisen .

## Patentansprüche

1. Verfahren zur Analyse von Stoffen unter Verwendung von lebenden biologischen Materialien, **dadurch gekennzeichnet**, daß biologische Materialien als Indikatoren verwendet werden, deren photoakustisch meßbare Absorption sich unter dem Einfluß der zu analysierenden Stoffe verändert.

2. Verfahren nach Anspruch 1, **dadurch gekennzeichnet**, daß biologisches Material verwendet wird, dessen photoakustisch meßbare Absorption sich selektiv unter dem Einfluß einzelner Stoffe verändert.

3. Verfahren nach Anspruch 1, **dadurch gekennzeichnet**, daß biologisches Material verwendet wird, dessen photoakustisch meßbare Absorption sich selektiv unter dem Einfluß bestimmter Stoffgruppen verändert.

4. Verfahren nach mindestens einem der Ansprüche 1 bis 3, **dadurch gekennzeichnet**, daß in einem ersten Schritt der biologische Indikator ohne den zu analysierenden Stoff photoakustisch vermessen und das Ergebnis zwischengespeichert werden, daß in einem zweiten Schritt der biologische Indikator mit dem zu analysierenden Stoff versetzt wird, daß in einem weiteren Schritt der mit dem zu analysierenden Stoff versetzte Indikator photoakustisch vermessen wird und daß anschließend dieses Meßergebnis mit dem zwischengespeicherten Meßergebnis verglichen wird und daß das daraus resultierende Meßergebnis als Maß für die zu untersuchende Stoffmenge dient.

5. Verfahren nach Anspruch 1 bis 4, **dadurch gekennzeichnet**, daß als biologisches Material Rhodopsin-Moleküle, Hämoglobin-Moleküle, pflanzliche Zellen, Flechten, Blätter, tierische Zellen, Mikroorganismen, Halobakterien, Hefen oder Pilze verwendet werden.

6. Verfahren nach mindestens einem der Ansprüche 1 bis 5, **dadurch gekennzeichnet**, daß als pflanzliche Zellen einzellige Algen verwendet werden.

7. Verfahren nach mindestens einem der Ansprüche 1 bis 5, **dadurch gekennzeichnet**, daß als tierische Zellen Blutzellen verwendet werden.

8. Verfahren nach mindestens einem der Ansprüche 1 bis 5, **dadurch gekennzeichnet**, daß als Mikroorganismen Halobakterien oder Hefen verwendet werden.

9. Verfahren nach mindestens einem der Ansprüche 1 bis 8, **dadurch gekennzeichnet**, daß die Verfahrensschritte zur Auswahl (Screening) biologischer Materialien verwendet werden, indem ein Stoff bzw. eine Stoffgruppe in bekannter Konzentration und Menge mit dem biologischen Material in Kontakt gebracht wird und aus der Reaktion des biologischen Materials auf dieses geschlossen wird.

10. Meßzelle zur Durchführung des Verfahrens nach mindestens einem der Ansprüche 1 bis 9, **dadurch gekennzeichnet**, daß sie zweiteilig ist und aus einem Unter- und einem Oberteil besteht, daß das Unterteil eine flache Schale (1) ist, die sich zur Züchtung des biologischen Materials eignet, daß das Oberteil (3) aus optisch durchsichtigem Glas besteht, einen Einfüll- und einen Austrittsstutzen (4, 5) hat, sowie eine Öffnung (6) für das akustische Ankoppeln eines photoakustischen Sensors aufweist.

11. Meßzelle nach Anspruch 10, **dadurch gekennzeichnet**, daß Ober- und Unterteil (1, 3) zur Gewährleistung der erforderlichen Dichtigkeit eingeschliffen und der Einfüll- und der Austrittsstutzen (4, 5) gasdicht verschließbar sind, sowie die Unterkante des Einfüllstutzens über die Unterkante des Oberteiles (3) in den Probenraum reicht.

12. Meßzelle nach Anspruch 10 und 11, **dadurch gekennzeichnet**, daß mindestens der Verschluß eines Stutzens, vorzugsweise des Einfüllstutzens, zur definierten Einleitung des Schadstoffes während des gesamten Analysenvorganges zu öffnen ist.
